# FASCICULE DE BREVET EUROPEEN

(11) **EP 0 714 882 B1**
(45) Date de publication et mention de la délivrance du brevet: **16.04.1997**
(21) Numéro de dépôt: 95402263.8
(22) Date de dépôt: 09.10.1995
(51) Int. Cl.: C07C 213/00, C07C 215/10, C07C 231/02

(54) **Procédé de préparation de 2-amino-alcane-1,3 diols**
Verfahren zur Herstellung von 2-Aminoalkan-1,3-diolen
Process for the preparation of 2-amino-alkan-1,3-dioles

(30) Priorité: 30.11.1994 FR 9414380
(43) Date de publication de la demande: 05.06.1996
(73) Titulaire: L'OREAL, 75008 Paris (FR)
(72) Inventeur: Semeria, Didier, F-77181 Courtry (FR); Philippe, Michel, F-91320 Wissous (FR)
(74) Mandataire: Tezier Herman, Béatrice

(56) Documents cités:
- EP-A- 0 500 437
- JOURNAL OF THE CHEMICAL SOCIETY, 1951 LETCHWORTH GB, pages 2453-2456, G. I. GREGORY, T. MALKIN 'The sphingolipid field. Part I. Synthesis of racemic 2-amino-octadecane-1:3-diols'
- JOURNAL OF ORGANIC CHEMISTRY, vol. 16, 1951 EASTON US, pages 269-278, K. HAYES, G. GEVER 'The preparation of 1-(2-furyl)-2-amino-1,3-propandiol and derivatives'
- CHEMISTRY & INDUSTRY, 1952 pages 130-131, N. FISHER 'synthesis of (racemic) dihydrosphingosine'
- JOURNAL OF THE AMERICAN CHEMICAL SOCIETY, vol. 70, 1948 WASHINGTON, DC US, pages 3121-3125, H. ADKINS, H. R. BILLICA 'The hydrogenation of esters to alkohols at 25-150 degrees celcius'

## Description

L'invention a pour objet un nouveau procédé de préparation de précurseurs de la synthèse des céramides.

Les céramides, à l'état naturel, sont les composants principaux des couches lipidiques de l'épiderme. Ils sont utilisés en cosmétique sous forme naturelle ou synthétique dans des compositions destinées, entre autre, à réduire le dessèchement de la peau ou à conférer à celle-ci une meilleure élasticité ou encore destinées au traitement du cheveu.

Les céramides naturels sont généralement obtenus par extraction de la peau de porc, du cerveau de boeuf, de l'oeuf, des cellules du sang, des plantes (JP86/260008 ou JP87/120308).

Les inconvénients nombreux liés à ce type d'approvisionnement (fragilité, contamination, conservation, coût, etc.) ont fait que très tôt la voie de la synthèse chimique a été explorée.

De nombreux travaux ont été développés sur ces voies de synthèse (Hayes et Gever J. Org. Chem. 1951, 16, 269 ; Gregory et Malkin J. Chem. Soc., 1951, 2453-2456 ; Fisher, N., Chemistry and Industrie 1952, 130-131; Shapiro, D. Chemistry of sphingolipids, Hermann, Paris, 1969,26-34).

Hayes et Gever proposent une voie de synthèse directe à partir d'un méthyl-α-oximino furoylacétate en une étape de réduction par de l'aluminohydrure de lithium. Cependant cette voie ne conduit qu'à l'obtention d'un mélange de composés à longues chaînes aminées, duquel aucun composé purifié ne peut être extrait. Le rendement de cette réaction dont les auteurs eux-mêmes soulignent la faiblesse (2%) fait que cette méthode ne peut raisonnablement trouver une application industrielle.

Gregory et Malkin en 1951 ont abordé le domaine de la synthèse des sphingolipides, et des précurseurs de la synthèse des céramides que sont les 2-amino-alcane-1,3-diols.

Ils enseignent une voie de synthèse comportant de nombreuses étapes à partir d'un méthyl-2-oximino-3-oxoalcanoate qui par réductions successives aboutit à la synthèse de 2-amino-alcane-1,3-diols. Là encore cette voie, du fait du grand nombre d'étapes de synthèse et de purification des intermédiaires, aboutit à des résultats médiocres quant au rendement. De plus, la succession de ces étapes allonge considérablement le temps nécessaire à la préparation de ces composés.

Fisher en 1952 évoque la voie de synthèse préconisée par Hayes et Gever, précisant toutefois que, après une réaction de protection par benzoylation, sans détailler les réactions mises en oeuvre, il a pu obtenir par réduction directe, un 2-amino-alcane-1,3-diol tribenzoylé. Ce composé une fois débenzoylé pourrait conduire à un 2-amino-alcane-1,3-diol.

Shapiro en 1969 reprend ces voies de synthèse en améliorant les étapes de protection des fonctions sensibles, en particulier l'amine. Cette voie de synthèse multi-étape est celle qui est depuis lors utilisée pour la synthèse des céramides et de leurs précurseurs. (FR 2673179).

Comme on peut aisément le comprendre de ce qui précède, l'obtention des précurseurs de céramides, à savoir les 2-amino-alcane-1,3-diols, si elle apparaît relativement bien décrite, nécessite de nombreuses réactions successives. Ceci a pour effet de rendre la synthèse des céramides longue et coûteuse.

Cette succession de réactions a également pour effet de réduire considérablement les rendements, ce qui participe à l'élévation du prix de revient de ces composés.

La demanderesse a donc recherché à améliorer les voies de synthèse des céramides et en particulier de leurs précurseurs, les 2-amino-alcane-1,3-diols, sans devoir protéger puis déprotéger les diverses fonctions.

Après de longues recherches et de manière surprenante et inattendue, contrairement à ce que l'art antérieur enseigne de manière constante depuis de longues années, la demanderesse a découvert que la voie de synthèse directe à partir d'un alkyl-2-oximino-3-oxoalcanoate en une seule étape de réduction peut conduire à un 2-amino-alcane-1,3-diol.

Supprimant de nombreuses étapes, l'invention permet un gain de temps non négligeable et un rendement de la synthèse très amélioré, qui industriellement diminuent le prix de revient des céramides synthétisés à partir du composé obtenu par le procédé revendiqué.

Ainsi, l'invention a pour objet un procédé de préparation de 2-amino-alcane-1,3-diol, caractérisé par une réduction en une seule étape des fonctions réductibles d'un alkyl-2-oximino-3-oxoalcanoate de formule (I) :

R₁-CO-CNOH-COOR₂ (I)

dans laquelle R₁ et R₂ représentent un radical alkyle linéaire ou ramifié, saturé ou insaturé,
en présence d'au moins un hydrure, dans un solvant, sous atmosphère inerte, et à une température initiale contrôlée.

Selon un mode préférentiel de réalisation du procédé, R₁ a de 5 à 29 atomes de carbone et R₂ a de 1 à 5 atomes de carbone. De manière avantageuse R₁ a de 11 à 21 atomes de carbone.

Selon un mode préféré de l'invention, R₂ est un radical méthyle ou éthyle.

Le procédé selon l'invention permet la synthèse en une étape des 2-amino-alcane-1,3-diols répondant à la formule (II):

R₁-CHOH-CHNH₂-CH₂OH (II)

ou des sels correspondants.

Le composé de formule (II) se présente après synthèse, sous la forme d'un mélange D,L-érythro/thréo. Ce mélange est en général, dans des proportions érythro/thréo allant de 90/10 à 20/80 et de préférence est de 85/15 à 35/65.

La réaction s'effectue dans un solvant. Ce solvant est de préférence anhydre et inerte vis à vis des réactifs présents. Par exemple on peut citer comme solvant le toluène, l'heptane, le tetrahydrofurane, le tertiobutylméthyléther, ou encore l'éther isopropylique. De manière préférentielle on utilise le tertiobutylméthyléther.

La réaction est réalisée sous atmosphère inerte, afin d'éviter la destruction du réactif par de l'air ou de l'eau. Pour cela on utilise généralement l'azote ou l'argon. De manière préférentielle on réalise la réaction sous atmosphère composée d'azote.

Par température initiale contrôlée, on entend que la réaction de réduction s'initie à une température allant de -10°C. à la température ambiante (environ 20°C). De préférence la réaction est initiée à 0°C.

La réaction peut se poursuivre à toute température comprise entre -10°C et la température de reflux du solvant utilisé. Avantageusement, la réaction est réalisée à la température de reflux du solvant utilisé.

Parmi les hydrures utilisables selon l'invention, on peut citer le borohydrure de lithium (LiBH₄), l'aluminohydrure de lithium (LiAlH₄), l'alluminohydrure (AlH₃), ou encore le bis-(2-méthoxy-éthoxy)-dihydroaluminate de sodium (RedAl™ vendu par la société Aldrich, ou vitride vendu par la société Hexcel). De manière préférentielle, on utilise le bis-(2-méthoxy-éthoxy)-dihydroaluminate de sodium.

En fin de réaction le produit synthétisé peut être sous forme de complexes d'aluminium. Avantageusement pour limiter la formation de ces complexes, des quantités minimales d'hydrure sont utilisées.

Ainsi l'hydrure est généralement présent dans la réaction à une concentration allant de 2 à 6 équivalents-molaires par rapport à l'alkyl-2-oximino-3-oxoalcanoate. De préférence on utilise une concentration allant de 2 à 4 équivalents-molaires par rapport à l'alkyl-2-oximino-3-oxoalcanoate.

A la fin de la réaction, marquée par la disparition des produits de départ, pour détruire les complexes éventuellement formés, on amène préférentiellement le pH du milieu réactionnel à une valeur inférieure à 2 ou supérieure à 11, par addition au milieu réactionnel d'acide ou de base aqueux.

Préférentiellement, le pH du milieu réactionnel est voisin de 1 ou voisin de 12. Ainsi, de préférence on utilise de l'acide chlorhydrique ou de la soude respectivement.

Les alkyl-2-oximino-3-oxoalcanoates utilisés dans les réactions selon l'invention peuvent être préparés selon les techniques couramment utilisées, notamment en faisant réagir un alkyl-3-oxoalcanoate avec un nitrite d'alkyle en milieu anhydre acidifié par de l'acide chlorhydrique gazeux. Préférentiellement on fait réagir un alkyl-3-oxoalcanoate avec un nitrite d'alkyle en présence d'une solution aqueuse acide, en quantité catalytique, comme par exemple de l'acide chlorhydrique concentré.

Par quantité catalytique on entend de 0,05 à 0,8 équivalent molaire d'acide et de préférence de 0,1 à 0,6 équivalent molaire d'acide.

Par nitrite d'alkyle, on entend un composé ayant pour formule R₃ONO, dans laquelle R₃ représente un radical alkyle ayant de 2 à 6 carbones, comme par exemple le nitrite de butyle.

Le composé obtenu selon l'invention peut être utilisé dans les procédés de synthèse de céramides comme ceux décrits par Shapiro, D. (Chemistry of sphingolipids, Hermann, Paris, 1969,26-34).

Ainsi, par exemple il est possible de préparer le céramide recherché, par acylation de la fonction amine d'un 2-amino-alcane-1,3-diol par un chlorure d'acide, par un anhydride, par un ester de paranitrophénol, par un ester de succinimide, par un ester de dicyclohexylcarbodiimide, par un ester d'alkyle inférieur, ou par un azolide comme notamment un imidazolide ou un pyrazolide, en milieu anhydre ou dans un solvant tel que le tétrahydrofuranne, la pyridine, le diméthylformamide ou le dichlorométhane. Avantageusement l'anhydride est un anhydride mixte, l'ester d'alkyle inférieur est un ester de méthyle ou d'éthyle et l'azolide est un imidazolide ou un pyrazolide.

On va donner maintenant, sans effets limitatifs, des exemples de synthèse selon l'invention.

### Exemple 1 : Synthèse du 2-amino-octadécane-1,3-diol:

1 mole d'oxo-3-octadécanoate de méthyle est dissoute dans de l'acétate d'éthyle. A cette solution on ajoute 1,05 moles de nitrite de butyle, puis lentement à température ambiante, 33 grammes d'acide chlorhydrique concentré jusqu'à la disparition totale du produit de départ.

Le mélange réactionnel est alors lavé à l'eau, puis la phase organique est évaporée ; l'huile résiduelle est redissoute dans de l'heptane puis recristallisée à froid, filtrée, lavée, et séché. On obtient ainsi du 2-oximino-3-oxo-octadécanoate de méthyle, avec un rendement de 80%. L'analyse de ce produit le montre conforme à la structure attendue.

Sous azote, on mélange 4 môles d'une solution de RedAl™ (70% en poids dans du toluène) dans du tertiobutylméthyléther. La solution est ensuite refroidie à la température de 0°C et on ajoute lentement 1 môle de 2-oximino-3-oxo-octadécanoate de méthyle. Le milieu réactionnel est alors chauffé progressivement jusqu'à 50°C. A la fin de la réaction, marquée par la disparition des produits de départ, le milieu réactionnel est hydrolysé à froid par une solution de soude. La phase aqueuse est décantée et la phase organique relavée par de l'eau, puis séchée.

Après refroidissement de la phase organique, le précipité est filtré et séché. On obtient du 2-amino-octadécane-1,3-diol sous la forme d'une poudre blanche, avec un rendement de 80%.

### Analyse :

pF : 81°C
RMN ¹³C : conforme à la structure : % Erythro/Thréo : 54/46
Analyse élémentaire : correcte.

## Revendications

1. Procédé de préparation de 2-amino-alcane-1,3-diol, ou des sels correspondants, caractérisé par le fait que l'on fait réagir en une seule étape un alkyl-2-oximino-3-oxoalcanoate de formule (I) :
R₁-CO-CNOH-COOR₂ (I)
dans laquelle R₁ et R₂ représentent un radical alkyle linéaire ou ramifié, saturé ou insaturé,
en présence d'au moins un hydrure, dans un solvant, sous atmosphère inerte, et à une température initiale contrôlée allant de -10°C à la température ambiante.

2. Procédé selon la revendication 1, dans lequel R₁ représente un radical alkyle ayant de 5 à 29 atomes de carbone.

3. Procédé selon la revendication 2, dans lequel R₁ représente un radical alkyle ayant de 11 à 21 atomes de carbone.

4. Procédé selon l'une quelconque des revendications précédentes, dans lequel R₂ représente un radical alkyle ayant de 1 à 5 atomes de carbone.

5. Procédé selon la revendication 4, dans lequel R₂ représente un radical méthyle ou éthyle.

6. Procédé selon l'une quelconque des revendications précédentes, dans lequel le solvant est un solvant inerte vis à vis des réactifs.

7. Procédé selon l'une quelconque des revendications précédentes, dans lequel le solvant est un solvant anhydre.

8. Procédé selon l'une quelconque des revendications précédentes, dans lequel le solvant est choisi parmi le toluène, l'heptane, le tetrahydrofurane, le tertiobutylméthyléther, ou encore l'éther isopropylique.

9. Procédé selon l'une quelconque des revendications précédentes, caractérisé par le fait que l'on travaille en atmosphère inerte.

10. Procédé selon la revendication 9, dans lequel l'atmosphère inerte est composée d'azote ou d'argon.

11. Procédé selon la revendication 10, dans lequel l'atmosphère inerte est composée d'azote.

12. Procédé selon l'une quelconque des revendications précédentes, dans lequel l'hydrure est choisi parmi le borohydrure de lithium (LiBH₄), l'aluminohydrure de lithium (LiAlH₄), l'alluminohydrure (AlH₃), ou encore le bis-(2-méthoxy-éthoxy)-dihydroaluminate de sodium (RedAl™ ou vitride).

13. Procédé selon la revendication 12, dans lequel l'hydrure est du bis-(2-méthoxyéthoxy)-dihydroaluminate de sodium.

14. Procédé selon l'une quelconque des revendications précédentes, dans lequel l'hydrure est à une concentration allant de 2 à 6 équivalents-molaires par rapport à l'alkyl-2-oximino-3-oxoalcanoate.

15. Procédé selon l'une quelconque des revendications 1 à 14, dans lequel l'hydrure est à une concentration allant de 2 à 4 équivalents-molaires par rapport à l'alkyl-2-oximino-3-oxoalcanoate.

16. Procédé selon l'une quelconque des revendications précédentes, dans lequel la réaction s'effectue à une température initiale contrôlée, puis se poursuit à toute température comprise entre -10°C et celle du reflux du solvant.

17. Procédé selon l'une quelconque des revendications 1 à 16, dans lequel la réaction une fois initiée, se poursuit à la température du reflux du solvant.

18. Procédé selon l'une quelconque des revendications 1 à 17, dans lequel la réaction s'initie à une température de 0°C.

19. Procédé selon l'une quelconque des revendications précédentes, dans lequel à la fin de la réaction le milieu réactionnel est amené à un pH inférieur à 2 ou supérieur à 11.

20. Procédé selon la revendication 19, dans lequel le pH du milieu réactionnel est voisin de 1.

21. Procédé selon la revendication 19, dans lequel le pH du milieu réactionnel est voisin de 12.

22. Procédé selon l'une quelconque des revendications 19 ou 20 dans lequel on utilise de l'acide chlorhydrique pour ajuster le pH.

23. Procédé selon l'une quelconque des revendications 19 ou 21 dans lequel on utilise de la soude pour ajuster le pH.

24. Procédé selon l'une quelconque des revendications précédentes, caractérisé par le fait que l'alkyl-2-oximino-3-oxoalcanoate de formule (I) est obtenu par réaction d'un alkyl-3-oxoalcanoate avec un nitrite d'alkyle, en milieu anhydre, acidifié par de l'acide chlorhydrique gazeux.

25. Procédé selon l'une quelconque des revendications 1 à 23, caractérisé par le fait que l'alkyl-2-oximino-3-oxoalcanoate de formule (I) est obtenu par réaction d'un alkyl-3-oxoalcanoate avec un nitrite d'alkyle en présence d'une solution en quantité catalytique d'acide chlorhydrique concentré.

26. Procédé de préparation de céramides, caractérisé en ce que l'on met en oeuvre le procédé selon l'une quelconque des revendications précédentes, l'on acyle la fonction amine du 2-amino-alcane-1,3-diol ainsi obtenu par un chlorure d'acide, par un anhydride, par un ester de paranitrophénol, par un ester de succinimide, par un ester de dicyclohexylcarbodiimide, par un ester d'alkyle inférieur, ou par un azolide comme notamment un imidazolide ou un pyrazolide, en milieu anhydre ou dans un solvant tel que le tétrahydrofuranne, la pyridine, le diméthylformamide ou le dichlorométhane.

27. Procédé de préparation de céramides selon la revendication 26, caractérisé en ce que l'anhydride est un anhydride mixte, l'ester d'alkyle inférieur est un ester de méthyle ou d'éthyle et l'azolide est un imidazolide ou un pyrazolide.

## Claims

1. Process for the preparation of 2-aminoalkane-1,3-diol or corresponding salts, characterized in that an alkyl 2-oximino-3-oxoalkanoate of formula (I):
R₁-CO-CNOH-COOR₂ (I)
in which R₁ and R₂ represent a saturated or unsaturated linear or branched alkyl radical,
is reacted in a single step in the presence of at least one hydride, in a solvent, under inert atmosphere, and at a controlled initial temperature ranging from -10°C to room temperature.

2. Process according to Claim 1, in which R₁ represents an alkyl radical having from 5 to 29 carbon atoms.

3. Process according to Claim 2, in which R₁ represents an alkyl radical having from 11 to 21 carbon atoms.

4. Process according to any one of the preceding claims, in which R₂ represents an alkyl radical having from 1 to 5 carbon atoms.

5. Process according to Claim 4, in which R₂ represents a methyl or ethyl radical.

6. Process according to any one of the preceding claims, in which the solvent is a solvent which is inert with respect to the reactants.

7. Process according to any one of the preceding claims, in which the solvent is an anhydrous solvent.

8. Process according to any one of the preceding claims, in which the solvent is chosen from toluene, heptane, tetrahydrofuran, tert-butyl methyl ether and isopropyl ether.

9. Process according to any one of the preceding claims, characterized in that it is carried out under inert atmosphere.

10. Process according to Claim 9, in which the inert atmosphere is composed of nitrogen or argon.

11. Process according to Claim 10, in which the inert atmosphere is composed of nitrogen.

12. Process according to any one of the preceding claims, in which the hydride is chosen from lithium borohydride (LiBH₄), lithium aluminium hydride (LiAlH₄), aluminium hydride (AlH₃), and sodium bis (2-methoxyethoxy)dihydroaluminate (RedAl™ or vitride).

13. Process according to Claim 12, in which the hydride is sodium bis(2-methoxyethoxy)dihydroaluminate.

14. Process according to any one of the preceding claims, in which the hydride is at a concentration ranging from 2 to 6 molar equivalents relative to the alkyl 2-oximino-3-oxoalkanoate.

15. Process according to any one of Claims 1 to 14, in which the hydride is at a concentration ranging from 2 to 4 molar equivalents relative to the alkyl 2-oximino-3-oxoalkanoate.

16. Process according to any one of the preceding claims, in which the reaction is carried out at a controlled initial temperature, and then continues at any temperature between -10°C and the reflux temperature of the solvent.

17. Process according to any one of Claims 1 to 16, in which, once initiated, the reaction continues at the reflux temperature of the solvent.

18. Process according to any one of Claims 1 to 17, in which the reaction is initiated at a temperature of 0°C.

19. Process according to any one of the preceding claims, in which, at the end of the reaction, the reaction medium is brought to a pH below 2 or above 11.

20. Process according to Claim 19, in which the pH of the reaction medium is in the region of 1.

21. Process according to Claim 19, in which the pH of the reaction medium is in the region of 12.

22. Process according to either of Claims 19 and 20, in which hydrochloric acid is used to adjust the pH.

23. Process according to either of Claims 19 and 21, in which sodium hydroxide is used to adjust the pH.

24. Process according to any one of the preceding claims, characterized in that the alkyl 2-oximino-3-oxoalkanoate of formula (I) is obtained by reaction of an alkyl 3-oxoalkanoate with an alkyl nitrite, in anhydrous medium, acidified with gaseous hydrogen chloride.

25. Process according to any one of Claims 1 to 23, characterized in that the alkyl 2-oximino-3-oxoalkanoate of formula (I) is obtained by reaction of an alkyl 3-oxoalkanoate with an alkyl nitrite in the presence of a catalytic amount of concentrated hydrochloric acid solution.

26. Process for the preparation of ceramides, characterized in that the process according to any one of the preceding claims is carried out, the amine function of the 2-aminoalkane-1,3-diol thus obtained is acylated with an acid chloride, with an anhydride, with a paranitrophenyl ester, with a succinimide ester, with a dicyclohexylcarbodiimide ester, with a lower alkyl ester or with an azolide such as, in particular, an imidazolide or a pyrazolide, in anhydrous medium or in a solvent such as tetrahydrofuran, pyridine, dimethylformamide or dichloromethane.

27. Process for the preparation of ceramides according to Claim 26, characterized in that the anhydride is a mixed anhydride, the lower alkyl ester is a methyl or ethyl ester and the azolide is an imidazolide or a pyrazolide.

## Patentansprüche

1. Verfahren zur Herstellung von 2-Amino-alkan-1,3-diolen oder den entsprechenden Salzen,
dadurch gekennzeichnet, daß
in einem einzigen Schritt ein Alkyl-2-oximino-3-oxoalkanoat der Formel (I):
R₁-CO-CNOH-COOR₂ (I)
worin R₁ und R₂ eine geradkettige oder verzweigte, gesättigte oder ungesättigte Alkylgruppe bedeuten, in Gegenwart mindestens eines Hydrids in einem Lösungsmittel unter Inertgas bei einer kontrollierten Anlfangstemperatur von -10 °C bis Raumtemperatur umgesetzt wird.

2. Verfahren nach Anspruch 1, dadurch gekennzeichnet, daß R₁ eine Alkylgruppe mit 5 bis 29 Kohlenstoffatomen bedeutet.

3. Verfahren nach Anspruch 2, worin R₁ eine Alkylgruppe mit 11 bis 21 Kohlenstoffatomen bedeutet.

4. Verfahren nach einem der vorhergehenden Ansprüche, worin R₂ eine Alkylgruppe mit 1 bis 5 Kohlenstoffatomen bedeutet.

5. Verfahren nach Anspruch 4, worin R₂ eine Methyl- oder Ethylgruppe bedeutet.

6. Verfahren nach einem der vorhergehenden Ansprüche, worin das Lösungsmittel inert gegenüber den Reaktanten ist.

7. Verfahren nach einem der vorhergehenden Ansprüche, worin das Lösungsmittel ein wasserfreies Lösungsmittel ist.

8. Verfahren nach einem der vorhergehenden Ansprüche, worin das Lösungsmittel unter Toluol, Heptan, Tetrahydrofuran, *tert*.-Butylmethylether oder auch Isopropylether ausgewählt ist.

9. Verfahren nach einem der vorhergehenden Ansprüche, dadurch gekennzeichnet, daß unter Inertgas gearbeitet wird.

10. Verfahren nach Anspruch 9, worin das Inertgas Stickstoff oder Argon ist.

11. Verfahren nach Anspruch 10, worin das Inertgas Stickstoff ist.

12. Verfahren nach einem der vorhergehenden Ansprüche, worin das Hydrid unter Lithiumborhydrid (LiBH₄), Lithiumaluminiumhydrid (LiAlH₄), Aluminiumhydrid (AlH₃) oder auch Natrium-bis-(2-methoxy-ethoxy)-dihydroaluminat (RedAl® oder Vitride) ausgewählt ist.

13. Verfahren nach Anspruch 12, worin das Hydrid Natrium-bis-(2-methoxy-ethoxy)-dihydroaluminat ist.

14. Verfahren nach einem der vorhergehenden Ansprüche, worin das Hydrid in einer Konzentration von 2 bis 6 mol pro Mol Alkyl-2-oximino-3-oxoalkanoat, vorliegt.

15. Verfahren nach einem der Ansprüche 1 bis 14, worin das Hydrid in einer Konzentration von 2 bis 4 mol pro Mol Alkyl-2-oximino-3-oxoalkanoat, vorliegt.

16. Verfahren nach einem der vorhergehenden Ansprüche, worin die Reaktion bei einer kontrollierten Anfangstemperatur durchgeführt wird, worauf die Reaktion bei einer beliebigen Temperatur von -10 °C bis zur Rückflußtemperatur des Lösungsmittels abläuft.

17. Verfahren nach einem der Ansprüche 1 bis 16, worin die einmal gestartete Reaktion bei der Rückflußtemperatur des Lösungsmittels abläuft.

18. Verfahren nach einem der Ansprüche 1 bis 17, worin die Reaktion bei einer Temperatur von 0 °C gestartet wird.

19. Verfahren nach einem der vorhergehenden Ansprüche, worin am Ende der Reaktion der pH-Wert des Reaktionsmediums auf einen Wert unter 2 oder über 11 eingestellt wird.

20. Verfahren nach Anspruch 19, worin der PH-Wert des Reaktionsmediums in der Nähe von 1 liegt.

21. Verfahren nach Anspruch 19, worin der pH-Wert des Reaktionsmediums in der Nähe von 12 liegt.

22. Verfahren nach einem der Ansprüche 19 oder 20, worin Salzsäure zur Einstellung des pH-Werts verwendet wird.

23. Verfahren nach einem der Ansprüche 19 oder 21, worin Natriumhydroxid zur Einstellung des pH-Werts verwendet wird.

24. Verfahren nach einem der vorhergehenden Ansprüche, dadurch gekennzeichnet, daß das Alkyl-2-oximino-3-oxoalkanoat der Formel (I) durch Umsetzung eines Alkyl-3-oxoalkanoats mit einem Alkylnitrit in einem wasserfreien Medium, das durch Salzsäuregas angesäuert ist, hergestellt wird.

25. Verfahren nach einem der Ansprüche 1 bis 23, dadurch gekennzeichnet, daß das Alkyl-2-oximino-3-oxoalkanoat der Formel (I) durch Umsetzung eines Alkyl-3-oxoalkanoats mit einem Alkylnitrit in Gegenwart einer Lösung hergestellt wird, die eine katalytische Menge konzentrierter Salzsäure enthält.

26. Verfahren zur Herstellung von Ceramiden, dadurch gekennzeichnet, daß das Verfahren nach einem der vorhergehenden Ansprüche angewandt wird, und die Aminogruppe des so hergestellten 2-Amino-alkan-1,3-diols mit einem Säurechlorid, einem Anhydrid, einem p-Nitrophenolester, einem Succinimidester, einem Dicyclohexylcarbodiimidester, einem niederen Alkylester oder einem Azolid, wie insbesondere einem Imidazolid oder einem Pyrazolid, in einem wasserfreien Medium oder einem Lösungsmittel, wie Tetrahydrofuran, Pyridin, Dimethylformamid oder Dichlormethan, acyliert wird.

27. Verfahren zur Herstellung von Ceramiden nach Anspruch 26, dadurch gekennzeichnet, daß das Anhydrid ein gemischtes Anhydrid, der niedere Alkylester ein Methyl- oder Ethylester und das Azolid ein Imidazolid oder ein Pyrazolid ist.
